(19) Europäisches Patentamt
European Patent Office
Office européen des brevets

(11) Veröffentlichungsnummer: **0 143 881**
**A2**

(12) # EUROPÄISCHE PATENTANMELDUNG

(21) Anmeldenummer: 84107902.3

(22) Anmeldetag: 06.07.84

(51) Int. Cl.[4]: C 07 K 5/06
//C07K1/08

(30) Priorität: 07.09.83 CH 4888/83
06.02.84 ES 529469

(43) Veröffentlichungstag der Anmeldung:
12.06.85 Patentblatt 85/24

(84) Benannte Vertragsstaaten:
AT BE CH DE FR GB IT LI LU NL SE

(71) Anmelder: GEMA S.A.
Via Augusta 158
Barcelona 6(ES)

(72) Erfinder: Palomo Coll, Alberto
Dr. Carulla 10
Barcelona(ES)

(72) Erfinder: Cabre Castellvi, Juan
Cardena 190
Barcelona(ES)

(72) Erfinder: Jackson, Barry, Dr.
Rathausstrasse 12
Visp (Canton Wallis)(CH)

(74) Vertreter: von Füner, Alexander, Dr. et al,
Patentanwälte v. Füner, Ebbinghaus, Finck Mariahilfplatz 2 & 3
D-8000 München 90(DE)

(54) **Verfahren zur Herstellung eines alpha-L-Aspartyl-L-Phenylalanin-alkylesters.**

(57) Vorgeschlagen wird ein Verfahren zur Herstellung von α-L-Aspartyl-L-phenylalanin-alkylestern durch Umsetzung von Asparaginsäure mit einem Alkalihydroxid oder einem organischem Amin, Weiterumsetzung des entstandenen Salzes mit einem $C_8$ bis $C_{20}$ Alkylacetoacetat oder einem $C_1$ bis $C_{20}$ Acetoacetamid, Ueberführung des erhaltenen DANE-Salzes mit einem Säurechlorid zum gemischten Anhydrid und Kondensation desselben mit Phenylalnin-alkylester zum gewünschten Endprodukt.

EP 0 143 881 A2

# Verfahren zur Herstellung eines α-L-Aspartyl-L-Phenylalanin-alkylesters

α-L-Aspartyl-L-phenylalanin-methylester wurde von R.H.Mazur, J.Am.Chem.Soc. 91 (1969) 2684 als Süssstoff entdeckt. Dieses Dipeptid zeigt eine Süsskraft, die 180 bis 200 mal stärker als diejenige von Saccharose ist. Es zeigen jedoch nur die α-L-Aspartyl-verbindungen Süsswirkung, die β-L-Aspartylverbindungen sind von bitterem Geschmack.
Daher hat es bis heute nicht an Versuchen gefehlt, einfache Verfahren zu entwickeln, die es zulassen, ausgehend von L-Phenylalanin-alkylestern und L-Asparaginsäure die gewünschten α-L-Aspartyl-L-phenylalanin-alkylester in reiner Form herzustellen.

So ist aus der EP-PS 0 035 047 bekannt, Asparaginsäure mit Acetylaceton oder mit niederen Acetessigsäurealkylestern in Gegenwart von Alkalihydroxiden oder von Aminen und Alkalihydroxiden zu den sogenannten DANE-Salzen umzusetzen und diese in einem weiteren Schritt zu einem inneren Anhydrid und schlussendlich mit Phenylalanin-methylester zum Zielprodukt umzusetzen. Nachteilig bei dieser Methode ist aber die 10 bis 20 Stunden dauernde Reaktionszeit für die Aktivierung des DANE-Salzes zum inneren Anhydrid. Des weiteren sind die isolierten Ausbeuten dieser Methode durch die aufwendige Aufarbeitung und Reinigungsprozedur mit 40 bis 51% eher bescheiden.

Ein weiteres Verfahren beschreibt die EP-PS 0 048 345, welches auf ähnliche Weise wie die EP-PS 0 035 047 mit niederen Acetessigsäurealkylestern die DANE-Salze herstellt, dieses mit Chlorameisensäureester zum gemischten Anhydrid und schlussendlich mit Phenylalanin-methylester zum Endprodukt umsetzt. Bei dieser Methode konnte zwar die Reaktionszeit für die Aktivierung verkürzt werden, nachteilig und nicht befriedigend ist aber neben der Anwendung sehr tiefer Reaktionstemperaturen von -30 bis -70°C wiederum die Aufarbeitung und Reinigung, die nur durch äusserst aufwendige und technisch nicht denkbare Säulenchromatographie gelöst werden kann.

Aufgabe vorliegender Erfindung ist es daher, ein Verfahren zur Herstellung eines α-L-Aspartyl-L-phenylalanin-alkylesters vorzuschlagen, das es gegenüber dem Stand der Technik möglich macht, den α-L-Aspartyl-L-phenylalanin-alkylester in kurzer Zeit auf einfache, in technischem Massstab durchführbare Weise, in guter Ausbeute und hoher Reinheit zu gewinnen.
Dies wird gemäss Patentanspruch 1 erreicht, indem man L-Asparaginsäure mit einem alkoholischen Alkalihydroxid oder einem organischen Amin das entsprechende bivalente Salz der Asparaginsäure bildet, die freie Aminogruppe des Salzes darauffolgend zum Schutz der Aminogruppen in situ mit einem Alkylacetoacetat der allgemeinen Formel $CH_3$-CO-$CH_2$-COOR, in welcher R Alkylreste mit 8 bis 20 C-Atomen bedeutet, oder einem Acetoacetamid der allgemeinen Formel $CH_3$-CO-$CH_2$-CONR'R", in welcher R' Wasserstoff oder Alkylreste mit 1 bis 20 C-Atomen und R" Alkylreste mit 1 bis 20 C-Atomen bedeuten, umsetzt und dieses

Umsetzungsprodukt isoliert und anschliessend in einem unpolaren Lösungsmittel mit einem Säurechlorid in das Anhydrid überführt, welches zum Schluss mit einem L-Phenylalanin-alkylester zum Endprodukt umgesetzt wird.

Die Bildung der bivalenten Salze der Asparaginsäure erfolgt erfindungsgemäss mit einem organischen Amin oder einem Alkalihydroxid.
Als organische Amine gelangen zweckmässig tertiäre und sekundäre Amine, wie z.B. Diäthylamin oder Triäthylamin, vorzugsweise Triäthylamin, zur Anwendung.
Als Alkalihydroxid wird zweckmässig Lithium-, Natrium- oder Kaliumhydroxid, vorzugsweise Kaliumhydroxid, angewendet.
Die Salzbildung kann zweckmässig in einem alkoholischen Medium mit Methanol, Aethanol oder anderen niederen Alkoholen durchgeführt werden.

Im weiteren bedarf es für die spätere Bildung des Dipeptids des Schutzes der Aminogruppe der L-Asparaginsäure. Gemäss vorliegender Erfindung geschieht dies in situ nach der Bildung des bivalenten Salzes mit organischen Verbindungen, die sich leicht an die Aminogruppe anknüpfen lassen, aber sich auch nach der Kondensationsphase mit L-Phenylalanin-alkylester leicht, z.B. durch Aenderung des pH-Wertes, wieder abspalten lassen. Dazu gehören die Alkylacetoacetat-verbindungen der allgemeinen Formel $CH_3-CO-CH_2-COOR$, in welcher R Alkylreste mit 8 bis 20 C-Atomen bedeuten. Besonders bevorzugte Verbindungen sind

Octylacetoacetat, Dodecylacetoacetat, Tetradecylacetoacetat, Hexadecylacetoacetat oder Octadecylacetoacetat.

Ebenfalls zu diesem Zweck gut einsetzbar sind die Acetoacetamide der allgemeinen Formel $CH_3$-CO-$CH_2$-CONR'R", in welcher R' Wasserstoff oder Alkylreste mit 1 bis 20 C-Atomen und R" Alkylreste mit 1 bis 20 C-Atomen bedeuten. Besonders bevorzugte Verbindungen sind Acetessigsäurediäthylamid und Acetessigsäuredodecylamid.

Man erhält dadurch die entsprechenden Aminogruppen-geschützten bivalenten Salze der L-Asparaginsäure, auch DANE-Salze genannt.

Die Reaktion der $NH_2$-Gruppe der Asparaginsäure mit den höheren Alkylacetoacetaten oder den Acetoacetamiden erfolgt zweckmässig in organischen Lösungsmitteln, vorzugsweise in niederen Alkoholen.

Wird die Asparaginsäure gemäss Anspruch 1 mit organischen Aminen und nachfolgend mit den erfindungsgemässen Alkylacetoacetaten oder den Acetoacetamiden umgesetzt, entstehen über die Di-ammonium-asparaginsäuresalze die Di-ammonium-DANE-Salze. Diese werden zweckmässig vor der Weiterumsetzung mit einem Alkalihydroxid der vorgängig genannten Art in das Di-alkali-DANE-Salz umgewandelt.

Gegenüber der direkten Herstellung des Di-alkali-DANE-Salzes durch Umsetzen der Asparaginsäure mit Alkalihydroxid und

Weiterumsetzung mit den erfindungsgemässen Alkylacetoacetaten oder Acetoacetamiden hat obige Methode den Vorteil, dass die Gefahr der Umesterung durch die im Reaktionsmedium anwesenden Alkohole weitgehend verhindert wird.

Diese erfindungsgemässen DANE-Salze der allgemeinen Formeln gemäss Patentanspruch 11 oder 12 können auf übliche Weise, z.B. durch Filtration, isoliert werden oder nach Austausch des polaren Lösungsmittels durch ein unpolares Lösungsmittel in situ weiterverwendet werden.

Zur Vorbereitung der Peptidbindung wird die Carboxylgruppe des DANE-Salzes mit anorganischen oder organischen Säuren, wie z.B. HCl, $H_2SO_4$, $HNO_3$, Essigsäure, Ameisensäure, Trifluoressigsäure, allein oder in Verbindung mit Amiden, wie N'N-Dimethylformamid, Acetamid und N'N-Dimethylacetamid als Amid-Salze oder in Verbindung mit Aminen, wie Pyridin, teilneutralisiert.

Dieses teilneutralisierte DANE-Salz wird in der Folge in Gegenwart von 4-Picolin als Katalysator mit organischen Säurechloriden, wie Hexansäurechloriden, Pentansäurechloriden oder Benzoylchloriden, vorzugsweise mit Pivaloylchlorid zum Anhydrid umgesetzt. Dieses bildet die Voraussetzung für die folgende Umsetzung mit der Aminogruppe des L-Phenylalaninalkylesters.

Dieses Anhydrid wird anschliessend mit einem Alkylester des L-Phenylalanins umgesetzt.

Als Alkylester des Phenylalanins kommen zweckmässig die niederen Alkylester mit 1 bis 4 C-Atomen, vorzugsweise die Methylester, in Frage.

Die Umsetzung des DANE-Salzes zum gemischten Anhydrid und die Kondensationsreaktion wird erfindungsgemäss in unpolaren Lösungsmitteln durchgeführt. Dazu kommen zweckmässig die Chloralkane, wie sie Methylenchlorid und Chloroform darstellen, vorzugsweise Methylenchlorid, zur Anwendung.

Zwecks Freisetzung des gewünschten α-L-Aspartyl-L-phenylalanin- alkylesters wird die Reaktionsmischung angesäuert, worauf das α-Produkt als festes Salz der entsprechenden Säure in guter Ausbeute ausfällt und direkt abgetrennt werden kann.

Somit wird durch die erfindungsgemässen, aus langkettigen Alkylacetoacetaten oder Acetoacetamiden gebildeten DANE-Salze ein einfaches, im technischen Massstab durchführbares Verfahren ermöglicht, das es erlaubt, die Umsetzung zum α-L-Aspartyl-L-phenylalanin-alkylester in unpolaren Lösungsmitteln bei kurzer Reaktionszeit durchzuführen. Eine aufwendige Aufarbeitung erübrigt sich dadurch ebenfalls, da das gewünschte α-Produkt in unpolarem Lösungsmittel bei der Säurezugabe in einer hohen Reinheit und guter Ausbeute anfällt.

Vorteilhaft wird das erfindungsgemässe Verfahren so durchgeführt, dass die L-Asparaginsäure mit einem organischen Amin in Mengen von 1,0 bis 3,5 Mol pro Mol, vorzugsweise 1,8 bis

3,0 Mol Asparaginsäure in einem alkoholischen Medium zum Di-Ammoniumsalz der Asparaginsäure umgesetzt wird.
Darauffolgend kann zur Herstellung des DANE-Salzes das Alkylacetoacetat oder das Acetoacetamid zugegeben werden.
Im Falle des Alkylacetoacetats gelangen Mengen von zweckmässig 0,7 bis 2,5 Mol, vorzugsweise 0,9 bis 2,1 Mol pro Mol Asparaginsäure zur Anwendung.
Im Falle des Acetoacetamids gelangen Mengen von zweckmässig 0,7 bis 2,5 Mol, vorzugsweise 0,9 bis 2,1 Mol pro Mol Asparaginsäure zur Anwendung.

Die Umsetzung wird zweckmässig bei Temperaturen zwischen 0°C und 60°C, vorzugsweise zwischen 10 und 30°C und während zweckmässig 5 bis 25 Stunden durchgeführt.

Vorteilhaft wird nun durch Zugabe eines Alkalihydroxids in Mengen von zweckmässig 1,6 bis 2,5 Mol, vorzugsweise 1,7 bis 2,1 Mol, zur Reaktionslösung das Di-Alkali-DANE-Salz hergestellt.
Das DANE-Salz kann isoliert oder in situ für den nächsten Schritt eingesetzt werden.

Zur Teilneutralisation der Carboxylgruppen gelangen nun anorganische oder organische Säuren allein oder in Verbindung mit Amiden als Amidsalz oder in Verbindung mit Aminen in Mengen von zweckmässig 0,8 bis 1,2 Mol, vorzugsweise 0,95 bis 1,2 Mol pro Mol DANE-Salz zur Anwendung.

In Gegenwart von zweckmässig 0,03 bis 0,05 Mol, vorzugsweise 0,035 bis 0,045 Mol 4-Picolin pro Mol DANE-Salz wird mit 0,5 bis 2,0 Mol, vorzugsweise 0,8 bis 1,2 Mol Pivaloylchlorid pro Mol DANE-Salz das Anhydrid gebildet.

Die Aktivierung, d.h. die Zeit für die Teilneutralisation und die Bildung des gemischten Anhydrids liegt zweckmässig zwischen 15 und 200 Minuten, vorteilhaft zwischen 30 und 100 Minuten. Das Anhydrid bildet die Voraussetzung zur Peptidbildung mit dem Phenylalaninalkylester, der in Mengen von zweckmässig 0,4 bis 1,2 Mol, vorzugsweise 0,6 bis 1,0 Mol, zugesetzt wird.

Die Umsetzung des DANE-Salzes zum Endprodukt wird in unpolaren Lösungsmitteln, wie Chloroform und Methylenchlorid, vorteilhaft in Methylenchlorid, durchgeführt.

Es wird bei Temperaturen zwischen -25 und +25°C, vorzugsweise zwischen -15 und +5°C, gearbeitet.

Zum Erhalten des α-L-Aspartyl-L-phenylalanin-methylesters wird der pH der Reaktionslösung durch Zugabe von z.B. Salzsäure auf -1 bis +4 gesenkt, worauf das α-Produkt als Salz ausfällt und durch Filtration abgetrennt werden kann.

Auf diese Weise erhält man Ausbeuten an α-L-Aspartyl-L-phenylalanin-alkylestersalz, die in der Regel zwischen 60 und 80% liegen.

Die Produkte liegen nach dem erfindungsgemässen Verfahren als Hydrochloride mit zwei Kristallwassern vor.

Die folgenden Beispiele erläutern das erfindungsgemässe Verfahren an der Herstellung des bevorzugten α-L-Aspartyl-L-phenylalanin-methylesterhydrochlorid-dihydrats.

Beispiel 1

Herstellung des (S)-N-(1-Methyl-2-dodecyloxycarbonylvinyl)-asparaginsäure-di-kaliumsalzes (DANE-DD-2K)

Eine Lösung von 66,6 g (S)-Asparaginsäure (0,50 Mol) und 125 ml Triäthylamin (0,90 Mol) in 500 ml Methanol wurde bei 20°C hergestellt, dann 273 g Acetessigsäuredodecylester (1,00 Mol) zugegeben und die Lösung bei 10°C über Nacht rühren gelassen. Die Lösung wurde dann auf 0°C abgekühlt und 63,5 g Kaliumhydroxid-Plätzchen (0,97 Mol) langsam zugegeben, so dass die Temperatur bei 0°C blieb. Die klar-gelbe Lösung wurde weitere 60 Minuten gerührt. Die DANE-Salz-Lösung wurde dann über 30 Minuten in 3'500 ml Aceton zugetropft und die entstandene Suspension weitere 30 Minuten gerührt, dann abfiltriert, mit 500 ml Aceton gewaschen und 24 Stunden unter Vakuum (bis 0,1 Torr) bei 40°C getrocknet. Diese Arbeitsweise lieferte 212,2 g DANE-Salz.

Die Ausbeute, bezogen auf Asparaginsäure, betrug 90,4%. Produkt-NMR ($CD_3OD$) δ in ppm: 0,89, Triplett, 3H: J=7 ($CH_3$-alkyl); 1,31, breites Singlett, 18H (9 x $CH_2$); 1,57, Triplett, 2H, J=7, ($CH_2$-alkyl); 1,94, Singlett, 3H ($CH_3$-vinyl); 2,65, Quartett, 1H, J=17,5; 2,75, Quartett, 1H, J=17,7; 3,96, Triplett, 2H, J=7 ($-CH_2-O$); 4,21, Quartett, 1H, J=7,5.

Beispiel 2

Herstellung des (S)-N-(1-Methyl-2-octyloxycarbonylvinyl)-asparaginsäure-di-kaliumsalzes (DANE-Oc-2K)

Analog zu Beispiel 1 wurden 217 g Acetessigsäureoctylester (1,00 Mol) mit 66,6 g Asparaginsäure (0,50 Mol) und 125 ml Triäthylamin (0,90 Mol) in 500 ml Methanol umgesetzt. Die Reaktionslösung wurde dann bei 0°C mit 65,0 g Kaliumhydroxid (1,00 Mol) behandelt und das DANE-Salz durch Zutropfen der methanolischen Lösung in 3'000 ml Aceton, gefolgt durch Filtration, isoliert. Das Produkt wurde dann 24 Stunden bei 45°C unter Vakuum (bis 0,1 Torr) getrocknet und lieferte 181,8 g DANE-Salz.

Die Ausbeute, bezogen auf Asparaginsäure, betrug 88,1%.

Produkt-NMR ($CD_3OD$) $\delta$ in ppm:0,89, Triplett, 3H, J=7 ($CH_3$-alkyl); 1,32, breites Singlett, (5 x $CH_2$); 1,58, Triplett, 2H, J=7 ($CH_2$-alkyl); 1,95, Singlett, 3H ($CH_3$-vinyl); 2,63, Quartett, 1H, J=17; 2,78, Quartett, 1H, J=17,7; 3,96, Triplett,2H, J=7 (-$CH_2$-O-); 4,18, Quartett, 1H, J=7, (Vinyl-CH); 4,38, Singlett und -NH 9,10, Doublett.

Beispiel 3

Herstellung des (S)-N-(1-Methyl-2-diäthylaminocarbonylvinyl)-asparaginsäure-di-kaliumsalzes

13,1 g Kaliumhydroxid (0,20 Mol) wurden in 80 ml Methanol gelöst und dann 13,35 g Asparaginsäure (0,10 Mol) zugegeben und die Mischung bis 60°C aufgeheizt. Bei 60°C wurden 17,0 g Acetessigsäure-diäthylamid (0,107 Mol) addiert und die Lösung eine

Stunde bei 60°C gehalten, dann auf 20°C abgekühlt. Diese Lösung wurde zur Trockene eingedampft und der Rückstand in 400 ml Aceton aufgeschlämmt und eine Stunde bei 20°C gerührt.
Das Produkt wurde abfiltriert und 24 Stunden bei 25°C unter vermindertem Druck (12 Torr) getrocknet und lieferte 32,55 g DANE-Salz.
Die Ausbeute, bezogen auf Asparaginsäure, betrug 90,9%.
Produkt-NMR ($CD_3OD$) $\delta$ in ppm: 1,11, Triplett, 6H, J=7 ($CH_3$-alkyl); 1,94, Singlett, 3H ($CH_3$-vinyl); 2,62, Quartett, 1H, J=17; 3,31, Komplex, xH ($CH_2$-alkyl + $CH_3OD$); 4,14, breites Triplett, 1H, J ca. 7.

Die Beispiele 4 - 9 zeigen weitere DANE-Salz-Herstellungen entsprechend Beispiel 1 und 2, nur sind die Parameter, Amin zur Bildung des bivalenten Salzes, Acetoacetat oder Amid als Aminoschutzgruppe und das Alkalihydroxid zur Bildung des Dialkali-DANE-Salzes variiert worden:

| Beispiel | Amin f. die Bildung des DANE-Salzes | Acetoacetat oder Amid | Alkalihydroxid | Reakt. zeit | Ausbeute |
|---|---|---|---|---|---|
| 4 | Tetramethylpropylendiamin | Octylacetoacetat | NaOH | 16 h | 79 % |
| 5 | Diäthylamin | Octylacetoacetat | NaOH | 22 h | 85 % |
| 6 | Diazobicyclo (5,4,0)undec-7en | Dodecylacetoacetat | NaOH | 22 h | 83 % |
| 7 | N',N-N',N'-tetramethylguanidin | Hexadecylacetoacetat | KOH | 19 h | 91 % |

| | | | | | |
|---|---|---|---|---|---|
| 8 | Triäthylamin | Acetessigsäure-cyclohexylamid | NaOH | 22 h | 92 % |
| 9. | Triäthylamin | Octadecylaceto-acetat | LiOH | 6 h | 75 % |

Beispiel 10

Aspartame-hydrochlorid aus dem DANE-Salz; (S)-N-(1-Methyl-2-dodecyloxycarbonylvinyl)-Asparaginsäure-di-kaliumsalz (DANE-DD-2K)

14,13 g DANE-Salz 97,5% (0,030 Mol) wurden bei +5°C in 200 ml Methylenchlorid suspendiert. Zu dieser Suspension wurden zur Teilneutralisation 3,72 g Dimethylacetamid·Hydrochlorid 99,7% (0,030 Mol) zugegeben und mit 5 ml Methylenchlorid hineingewaschen. Diese Lösung wurde bei -10°C 15 Minuten gerührt. Dann wurden 0,15 g 4-Picolin (0,00115 Mol) und 3,3 g Pivalinsäurechlorid 99% (0,027 Mol) zugegeben, mit 5 ml Methylenchlorid hineingewaschen und bei -10°C 60 Minuten gerührt.
Zu dieser klaren Lösung wurden 3,65 g L- Phenylalanin-methylester 98,3% (0,020 Mol) in 25 ml Methylenchlorid gelöst und während 20 Minuten zugetropft. Diese Lösung wurde 1 Stunde bei -10°C gerührt.
Dann wurden 21 ml 1 N Salzsäure (0,021 Mol), nach weiteren 5 Minuten 2 ml konz. Salzsäure (0,020 Mol) und nach weiteren 5 Minuten 2,5 ml 4 N NaOH bis zu einem pH-Wert von 0,09 zugegeben.
Das Gemisch wurde 2 Stunden bei +1°C gerührt, abgenutscht, das feste Produkt in 200 ml n-Hexan aufgeschlämmt, dann abgenutscht

und bei 30°C unter Vakuum (20 mmHg) 15 Stunden getrocknet. Bezogen auf 100%iges Reinprodukt konnte eine Ausbeute von 4,80 g = 65,4% α-Aspartame· HCl·$2H_2O$, berechnet auf L-Phenylalanin-methylester, erhalten werden.

Die wässrige Phase enthielt noch 2,5% α-Aspartame·HCl·$2H_2O$, bezogen auf L-Phenylalanin-methylester.

Beispiel 11

Aspartame-hydrochlorid aus dem DANE-Salz; (S)-N-(1-Methyl-2-octyloxycarbonylvinyl)-asparaginsäure-di-kaliumsalz (DANE-OC-2K)

11,35 g DANE-Salz 93% (0,026 Mol) wurden bei +20°C in 100 ml Methylenchlorid suspendiert. Zu dieser Suspension wurden zur Teilneutralisation 3,22 g Dimethylamid·Hydrochlorid 100% (0,026 Mol) zugegeben und mit 2 ml Methylenchlorid hineingewaschen. Diese Suspension wurde auf -10°C gekühlt und 10 Minuten gerührt. Dann wurde 0,17 g 4-Picolin 100% (0,0013 Mol) und 3,0 g Pivalinsäurechlorid 99% (0,025 Mol) zugegeben, mit 3 ml Methylenchlorid hineingewaschen und 60 Minuten bei -10°C gerührt. Zu dieser klaren Lösung wurden 3,65 g L-Phenylalanin-methylester 99,8% (0,020 Mol), in 25 ml Methylenchlorid gelöst, während 15 Minuten getropft.

Das Reaktionsgemisch wurde 45 Minuten bei -10°C gerührt.

Dann wurden 21 ml 1 N Salzsäure (0,021 Mol), nach weiteren 3 Minuten 2,5 ml konz. Salzsäure (0,025 Mol) und nach weiteren 4 Minuten 3 ml 4 N NaOH bis zu einem pH-Wert von 0,05 zugegeben.

Das Gemisch wurde 60 Minuten bei 0°C gerührt, abgenutscht, das feste Produkt in 250 ml n-Hexan aufgeschlämmt und bei +30°C unter Vakuum (20 mmHg) 15 Stunden getrocknet.
Bezogen auf 100%iges Reinprodukt konnte eine Ausbeute von 4,60 g = 62,7% α-Aspartame·HCl·$2H_2O$, berechnet auf L-Phenylalanin-methylester, erhalten werden.
Aus der Mutterlauge konnten noch 3,4% α-Aspartam·HCl·$2H_2O$, bezogen auf L-Phenylalanin-methylester, gewonnen werden.

Beispiel 12

Aspartame-hydrochlorid aus dem DANE-Salz; (S)-N-(1-Methyl-2-dimethylaminocarbonylvinyl)-asparaginsäure-di-kaliumsalz

14,35 g DANE-Salz 96,9% (0,040 Mol) wurden bei +20°C in 150 ml Methylenchlorid suspendiert. Zu dieser Suspension wurden zur Teilneutralisation 4,96 g Dimethylacetamid·Hydrochlorid (0,020 Mol) zugegeben und mit 2 ml Methylenchlorid hineingewaschen. Diese Lösung wurde auf -10°C gekühlt und während 12 Minuten gekühlt. Dann wurde 0,17 g 4-Picolin·HCl 100% (0,013 Mol) und 3,66 g Pivalinsäurechlorid 99% (0,030 Mol) zugegeben, mit 2 ml Methylenchlorid hineingewaschen und 45 Minuten bei -10°C gerührt. Zu dieser Lösung wurden 3,65 g L-Phenylalanin-methylester 99,8% (0,020 Mol), in 25 ml Methylenchlorid gelöst, während 15 Minuten zugetropft. Das Reaktionsgemisch wurde während 60 Minuten bei -10°C gerührt.
Dann wurden 21 ml 1 N Salzsäure (0,021 Mol), nach weiteren 3 Minuten 4,2 ml konz. Salzsäure (0,042 Mol) und nach weiteren 3 ml 4 N NaOH bis zu einem pH-Wert von 0,12 zugegeben.

Das Gemisch wurde 90 Minuten bei +1/+2°C gerührt, abgenutscht, das feste Produkt in 200 ml n-Hexan aufgeschlämmt, filtriert und bei 30°C unter Vakuum (20 mmHg) 15 Stunden getrocknet. Bezogen auf 100%iges Reinprodukt konnte eine Ausbeute von 3,35 g = 45,7% α-Aspartame·HCl·$2H_2O$, berechnet auf L-Phenylalanin-methylester, erhalten werden.

Aus der Mutterlauge konnten noch 3,5% als α-Aspartame·HCl·$2H_2O$, bezogen auf L-Phenylalanin-methylester, gewonnen werden.

Beispiel 13

Aspartame-hydrochlorid aus dem DANE-Salz; (S)-N-(1-Methyl-2-octyloxycarbonylvinyl)-asparaginsäure-di-natriumsalz

(DANE-Oc-2Na)

8,29 g DANE-Salz (0,019 Mol) wurden bei +20 bis 22°C in 45 ml Methylenchlorid suspendiert. Zu dieser Suspension wurden bei -10 bis -15°C 2,57 g Pyridin·HCl (0,022 Mol) zugegeben und während 15 Minuten gehalten. Dann wurden 2 ml 1%ige 4-Picolin·HCl-Lösung und 2,44 g Pivalinsäurechlorid (0,02 Mol) zugegeben, mit 6 ml Methylenchlorid hineingewaschen und 15 Minuten bei 0 bis 2°C gerührt. Zu dieser klaren Lösung wurden 2,52 g L-Phenylalanin-methylester 98% (0,014 Mol), in 14 ml Methylenchlorid gelöst, während 1 bis 2 Minuten zugetropft. Das Reaktionsgemisch wurde 30 Minuten bei 0 bis -5°C gerührt.

Dann wurden 34 ml 1 N Salzsäure und nach weiteren 3 Minuten 3,91 ml konz. Salzsäure zugegeben. Das Gemisch wurde 60 Minuten bei 0°C gerührt, abgenutscht, das feste Produkt in 200 ml n-Hexan aufgeschlämmt und bei +30°C unter Vakuum (20 mmHg) 15 Stunden getrocknet.

Bezogen auf 100%iges Reinprodukt konnte eine Ausbeute von 3,95 g = 76,3% α-Aspartame·HCl·$2H_2O$, berechnet auf L-Phenylalanin-methylester, erhalten werden.

Beispiel 14

Aspartame-hydrochlorid aus dem DANE-Salz; (S)-N-(1-Methyl-2-isooctyloxycarbonylvinyl)-asparaginsäure-di-natriumsalz

(DANE-isooc-2Na)

8,35 g DANE-Salz (0,019 Mol) wurden bei +20 bis 22°C in 45 ml Methylenchlorid suspendiert. Zu dieser Suspension wurden bei -10 bis -15°C 2,74 g Dimethylacetamid-Hydrochlorid (0,022 Mol) zugegeben und während 10 Minuten gehalten. Dann wurden 2 ml 1%ige 4-Picolin·HCl-Lösung und 2,44 g Pivalinsäurechlorid (0,02 Mol) zugegeben, mit 6 ml Methylenchlorid hineingewaschen und bei 0 bis 5°C 30 Minuten gerührt. Zu dieser klaren Lösung wurden 2,52 g 98% L-Phenylalanin-methylester (0,014 Mol), in 14 ml Methylenchlorid gelöst, während 1 bis 2 Minuten zugegeben. Das Reaktionsgemisch wurde 45 Minuten bei 0 bis 5°C gerührt.

Dann wurde entsprechend Beispiel 13 hydrolisiert.

Nach Filtration und Trocknung wurden, bezogen auf 100%iges Reinprodukt, 3,72 g = 72,7%, α-Aspartam·HCl·$2H_2O$, berechnet auf L-Phenylalanin-methylester, isoliert.

Entsprechend den Beispielen 11 und 12, aber mit anderen Parametern, wurden die Beispiele 15 bis 29 durchgeführt.

| Beispiel | Ausgangs-DANE-Salz 0,019 Mol | Teilneutralisation Amid 0,022 Mol | Teilneutralisation Säure | Aktivierung | Reaktionszeit f.Halbneutralisation und Aktivierung | Umsetzung mit Pherylalaninmethylester (0,015 Mol) Reaktionstempertur | Ausbeute |
|---|---|---|---|---|---|---|---|
| 15 | DANE-Oc-2Na | DIMAC | $H_2SO_4$ | n.Beispiel 11 | 80 Min. | -10 bis 12°C | 75,0 % |
| 16 | " | MAC | $H_2SO_4$ | " | 75 Min. | 0°C | 68,5 % |
| 17 | " | —— | $H_2SO_4$ | " | 200 Min. | -10 bis 12°C | 68,0 % |
| 18 | " | TMH | $H_2SO_4$ | " | 105 Min. | -10 bis 12°C | 78,0 % |
| 19 | " | DMF | $H_2SO_4$ | " | 80 Min. | 0 bis 5°C | 73,0 % |
| 20 | " | DIMAC | $HNO_3$ | " | 120 Min. | 0 bis 5°C | 70,0 % |
| 21 | " | DIMAC | $CF_3COOH$ | " | 80 Min. | 0 bis 5°C | 48,0 % |
| 22 | " | DIMAC | $CF_3SO_3H$ | " | 75 Min. | -10 bis 12°C | 60,0 % |
| 23 | " | DIMAC | $CCl_2COOH$ | " | 65 Min. | 0 bis 5°C | 60,0 % |
| 24 | " | DIMAC | HCl | " | 120 Min. | -10 bis -15°C | 69,0 % |
| 25 | " | DMF | HCl | " | 90 Min. | -10 bis -12°C | 72,0 % |
| 26 | " | AC | HCl | " | 80 Min. | 0 bis -2°C | 69,0 % |
| 27 | DANE-DD-2K | DMAC | HCl | " | 70 Min. | 0 bis 5°C | 65,0 % |
| 28 | DANE-HD-2Na (Hexadecyl) | DIMAC | HCl | " | 45 Min. | 0 bis 5°C | 57,0 % |
| 29 | DANE-TD-2K | DIMAC | HCl | " | 70 Min. | 0 bis 2°C | 64,0 % |

AC= Acetamid/DIMAC= N'N-Dimethylacetamid/DMF= N'N-Dimethylformamid/MAC= N-Methylacetamid/TMH= N'N,N'N-Tetramethylharnst.

Patentansprüche

1. Verfahren zur Herstellung eines α-L-Aspartyl-L-phenylalanin-alkylesters, dadurch gekennzeichnet, dass L-Asparaginsäure mit einem alkoholischen Alkalihydroxid oder einem organischen Amin zur Bildung des entsprechenden bivalenten Salzes der Asparaginsäure umgesetzt wird, die freie Aminogruppe des Salzes darauffolgend zum Schutz der Aminogruppen in situ mit einem $C_8$ bis $C_{20}$ Alkylacetoacetat der allgemeinen Formel $CH_3$-CO-$CH_2$-COOR, in welcher R Alkylreste mit 8 bis 20 C-Atomen bedeutet, oder einem Acetoacetamid der allgemeinen Formel $CH_3$-CO-$CH_2$-CONR'R", in welcher R' Wasserstoff oder Alkylreste mit 1 bis 20 C-Atomen und R" Alkylreste mit 1 bis 20 C-Atomen bedeuten, umgesetzt wird, dieses Umsetzungsprodukt anschliessend in einem unpolaren Lösungsmittel mit einem Säurechlorid in das Anhydrid übergeführt wird und dieses mit einem Alkylester des L-Phenylalanins bei anschliessender Abspaltung der Schutzgruppen in das Endprodukt umgesetzt wird.

2. Verfahren nach Anspruch 1, dadurch gekennzeichnet, dass der Alkylrest des Alkylacetoacetats geradkettig oder verzweigt ist und 8 bis 20 C-Atome, vorzugsweise 8 bis 12 C-Atome, enthält.

3. Verfahren nach Ansprüchen 1 und 2, dadurch gekennzeichnet, dass man die Alkylacetoacetate in Mengen von 0,7 bis 2,5

Mol, vorzugsweise 0,9 bis 2,1 Mol pro Mol eingesetzte Asparaginsäure einsetzt.

4. Verfahren nach Ansprüchen 1 bis 3, dadurch gekennzeichnet, dass die Aminogruppe des Acetoacetamides unsubstituiert, einfach oder zweifach substituiert ist und dass man als Substituenten Alkylreste, geradkettig oder verzweigt, mit 1 bis 20 C-Atomen, vorzugsweise 2 bis 12 C-Atomen, einsetzt.

5. Verfahren nach Ansprüchen 1 bis 4, dadurch gekennzeichnet, dass man die Acetoacetamide in Mengen von 0,7 bis 2,5 Mol, vorzugsweise 0,9 bis 2,1 Mol pro Mol eingesetzte Asparaginsäure einsetzt.

6. Verfahren nach Ansprüchen 1 bis 5, dadurch gekennzeichnet, dass man zur Bildung des Anhydrids Pivaloylchlorid in Mengen von 0,5 bis 2,0 Mol, bezogen auf ein Mol Aminogruppen-geschütztes Umsetzungsprodukt, einsetzt.

7. Verfahren nach Ansprüchen 1 bis 6, dadurch gekennzeichnet, dass man als Alkylester des L-Phenylalanins, zweckmässig Methyl-, Aethyl-, Propyl- oder Butylester, vorzugsweise Methylester, in Mengen von 0,4 bis 1,2 Mol, bezogen auf ein Mol Aminogruppen-geschütztes Umsetzungsprodukt, anwendet.

8. Verfahren nach Ansprüchen 1 bis 7, dadurch gekennzeichnet, dass als organisches Amin zweckmässig sekundäres und tertiäres Amin, vorzugsweise tertiäres Amin in Mengen von 1,0 bis 3,5 Mol, vorzugsweise 1,8 bis 3,0 Mol, eingesetzt wird.

9. Verfahren nach Ansprüchen 1 bis 8, dadurch gekennzeichnet, dass wenn das Aminogruppen-geschützte Umsetzungsprodukt als Di-ammoniumsalz vorliegt, dieses mit Alkalihydroxid zum Di-alkalisalz umgesetzt wird.

10. Verfahren nach Ansprüchen 1 bis 9, dadurch gekennzeichnet, dass man als Alkalihydroxide Lithiumhydroxid, Natriumhydroxid oder Kaliumhydroxid, vorzugsweise aber Kaliumhydroxid in Mengen von 1,6 bis 2,5 Mol, vorzugsweise 1,7 bis 2,1 Mol pro Mol Asparaginsäure einsetzt.

11. Verbindungen der Formel

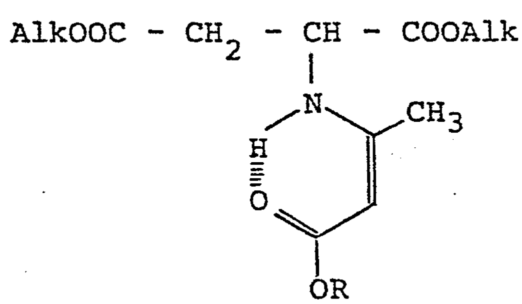

worin Alk = Li-, Na- oder K-Atome und R = Alkylreste, geradkettig oder verzweigt, mit 8 bis 20 C-Atomen, bedeuten.

12. Verbindungen der Formel

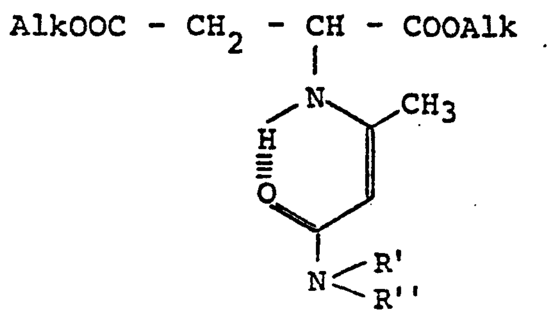

worin Alk = Li-, Na- oder K-Atome, R' = Wasserstoff oder Alkylreste, geradkettig oder verzweigt, mit 1 bis 20 C-Atomen, und R" Alkylreste, geradkettig oder verzweigt, mit 1 bis 20 C-Atomen, bedeuten.